# EUROPEAN PATENT SPECIFICATION

(11) **EP 2 724 683 B1**
(45) Date of publication and mention of the grant of the patent: **27.03.2019**
(21) Application number: 13190361.9
(22) Date of filing: 25.10.2013
(51) Int. Cl.: A61B 18/14, A61B 34/20, A61B 18/00

(54) **Irrigated ablation catheter with deformable head**
Bewässerter Ablationskatheter mit verformbarem Kopf
Cathéter d'ablation irrigué avec tête déformable

(30) Priority: 26.10.2012 US 201261718981 P; 12.09.2013 US 201314025233
(43) Date of publication of application: 30.04.2014
(60) Divisional application: 19153711.7
(73) Proprietor: Biosense Webster (Israel) Ltd., Yokneam 2066717 (IL)
(72) Inventor: Bar-Tal, Meir, 3224009 Haifa (IL)
(74) Representative: Carpmaels & Ransford LLP

(56) References cited:
- EP-A1- 1 679 034
- WO-A1-97/25917
- WO-A1-2007/055783
- US-A- 5 846 238
- US-A1- 2009 043 301
- US-B1- 6 923 805

## Description

### FIELD OF THE INVENTION

The present invention relates generally to invasive probes, and specifically to a probe with a deformable distal end.

### BACKGROUND

Various therapeutic procedures such as cardiac ablation use an invasive medical probe such as a catheter that is inserted into a patient's body. During an ablation procedure on a heart, there may be local overheating of the heart surface being ablated, as well as of the heart tissue underlying the surface. The surface overheating may be manifested as charring, and the overheating of the underlying tissue may cause other damage to the tissue, even leading to penetration of the tissue. To control the temperature of the surface and the underlying tissue, the region being ablated may be irrigated with an irrigation fluid, typically saline, in order to prevent charring.

In addition to the risk of charring, overheating of blood in the region being ablated may cause the formation of potentially dangerous blood clots, which can grow and potentially cause a heart attack or a stroke. While the irrigation may slightly reduce blood clot formation by cooling and diluting the blood, there is still a possibility of clotting.

A number of catheters with flexible tips have been described in the patent literature. For example, U.S. Patent 5,720,719, describes a catheter having a probe end that includes a malleable tube and a flexible tube. These elements are said to allow the probe end to conform to the curvature of the cavity inside a patient's body.

The description above is presented as a general overview of related art in this field and should not be construed as an admission that any of the information it contains constitutes prior art against the present patent application.

WO 97/25917 A1 discloses systems and methods for heating body tissue: place a multi-function structure having an exterior wall in contact with body tissue. The structure includes an array of electrically conducting electrode segments carried by the exterior wall. An electrically conductive network is coupled to the electrode segments, including at least one electrically conductive path individually coupled to each electrode segment. The systems and methods operate in a 1st mode during which the network is electrically conditioned to individually sense at each electrode segment local electrical events in tissue, such as electrical potentials, resistivity, or impedance. The systems and methods operate in a 2nd mode during which the network is electrically conditioned, based at least in part upon local electrical events sensed by the electrode segments, to couple at least two electrode segments together to simultaneously transmit electrical energy to heat or ablate a region of body tissue.

US5846238 discloses collapsible electrode assemblies.

### SUMMARY OF THE INVENTION

The invention is defined in appended independent claim 1, preferred embodiments are described in the dependent claims.

There is provided, in accordance with an embodiment of the present invention a medical probe, including a flexible insertion tube having a deformable distal end for insertion into a body cavity of a patient, the deformable distal end including a flexible and porous material configured to be brought into contact with tissue in the body cavity. The medical probe also includes a means for inflating the deformable distal end, and a channel contained within the insertion tube and configured to convey a fluid that irrigates the tissue through pores of the deformable distal end. The medical probe further includes an electrical conductor passing through the flexible insertion tube, terminating in the deformable distal end and configured to convey radio frequency (RF) energy to the tissue via the deformable distal end.

In some embodiments, the flexible and porous material may include a conductive material, the electrical conductor can be coupled to the flexible and porous material so as to convey the RF energy to the deformable distal end, and the RF energy can be conveyed to the tissue by the deformable distal end conveying the RF energy to the tissue.

In embodiments where the flexible and porous material may include a conductive material, the conductive material may include a fabric woven from strands of Nitinol. In additional embodiments where the flexible and porous material may include a conductive material, the conductive material can be configured to transfer a current, and the medical probe may further include a processor configured to determine a position of the distal end in response to an impedance to the current.

In further embodiments, the fluid may include a saline solution. In embodiments where the fluid includes a saline solution, the electrical conductor can convey the RF energy to the tissue by conveying the RF energy to the saline solution, and the saline solution conveying the RF energy to the tissue.

In some embodiments, the medical probe may include an intracardiac catheter, and the body cavity may include a chamber of a heart. In additional embodiments, the deformable distal end can be configured to conform to the tissue of the body cavity. In further embodiments, the insertion tube has a first diameter, and upon inflation of the deformable distal end, the deformable distal end has a second diameter greater than the first diameter. In supplementary embodiments, a contact area between the deformable distal end and the tissue can increase upon pressing the deformable distal end against the tissue.

In some examples of the disclosure, the means for inflating the deformable distal end may include conveying the fluid so as to generate a mechanical force sufficient to inflate the deformable distal end. According to the invention, the means for inflating the deformable distal end includes a wire frame protruding from a distal tip of the flexible insertion tube and covered by the deformable distal end, and a control wire, passing through the flexible insertion tube, coupled to the wire frame and configured to resize the wire frame. In the alternative embodiment including the wire frame, the electrical conductor terminates in the wire frame, and the wire frame is configured to convey the RF energy from the electrical conductor to the deformable distal end.

There is also disclosed, a method, including inserting a deformable distal end of a flexible insertion tube into a body cavity of a patient, the deformable distal end including a flexible and porous material configured to be brought into contact with tissue in the body cavity. The method also includes inflating the deformable distal end, and conveying a fluid through a channel contained within the flexible insertion tube so as to irrigate the tissue through pores of the deformable distal end. The method further includes conveying radio frequency (RF) energy to the tissue via the deformable distal end.

### BRIEF DESCRIPTION OF THE DRAWINGS

The disclosure is herein described, by way of example only, with reference to the accompanying drawings, wherein:
Figure 1 is a schematic, pictorial illustration of a medical system that includes an invasive probe having a deformable head;
Figure 2A is a schematic sectional view of the distal end of the probe;
Figures 2B and 2C are pictorial illustrations of a woven fabric used to construct a deformable head of the probe;
Figure 2D is a schematic pictorial illustration of the deformable head in contact with endocardial tissue of a heart;
Figure 3A is a schematic sectional view of the distal end of the probe, in accordance with an embodiment of the present invention;
Figure 3B is a schematic illustration of a wire frame extending from the distal end of the probe, in accordance with the embodiment of the present invention;
Figure 3C is a schematic pictorial illustration of the deformable head incorporating the wire frame and in contact with the endocardial tissue, in accordance with the embodiment of the present invention; and
Figures 4A-4C show heat maps that illustrate areas of contact between the deformable catheter head and the endocardial tissue, in accordance with an embodiment of the present invention.

### DETAILED DESCRIPTION OF EMBODIMENTS OVERVIEW

Catheters used in invasive cardiac procedures, such as intracardiac ablation for treatment of arrhythmias, typically have a rigid tip. When the tip is brought into contact with myocardial tissue at the proper angle and with sufficient force, the tissue conforms to the tip, affording good mechanical and electrical contact. The area of contact is typically limited by the tip size, however, and may be even smaller, depending on the angle of contact and other parameters.

Embodiments of the present invention provide an irrigated ablation catheter that has a deformable head. The deformable head increases the surface area that physically interfaces with the tissue and may also be useful in ensuring that the contact pressure between the catheter head and the tissue is roughly constant over the entire area of contact.

The catheter head can be fabricated from a porous cloth- like material, which may itself be flexible and/or conductive. In some embodiments, during an ablation procedure using the catheter, fluid, such as saline solution, can be forced through the porous material, so as to irrigate the area being ablated, as well as to irrigate the cloth-like material. (Irrigating the material ensures the integrity of the material, by keeping the material cool.) The fluid provides a means for delivering sufficient mechanical force to inflate the head, and to keep the head inflated during the procedure. Because the same saline solution can be for both irrigation and inflation, a separate inflating system is not required.

In examples, the cloth-like material covers an expandable wire frame that protrudes from a distal end of the catheter, and the size of the wire frame can be
managed via a control wire coupled to the frame. For example, an operator (e.g., a cardiologist) can expand the wire frame which provides a means to "inflate" the head by "pushing" the control wire toward the distal end. Likewise, the operator can contract the wire frame (and thereby "deflate" the head) by "pulling" the control wire away from the distal end. While examples herein describe a catheter head that can be resized via fluid pressure or a resizable wire frame, other methods of expanding and contracting the catheter head are possible.

Once in contact with the tissue, the catheter head conforms to the tissue, due to a mechanical response from the tissue and the flexibility of the head. The conformation with the tissue provides a larger interface surface compared with a rigid catheter tip.

During insertion, the catheter head need not be inflated and a sheath of the catheter can thus have a small diameter. After inflation the catheter head may be of a larger diameter than the sheath, providing a larger surface for ablation. Using this approach, a smaller-diameter ablation catheter can be used to treat ablation areas that would otherwise require a larger-diameter catheter for treatment. Having a larger ablation surface can support deeper ablation, such as for use in the left ventricle, or for use in forming scar tissue where a specific target location may not be known.

Although the disclosed embodiments relate specifically to intracardiac ablation, the principles of this invention may similarly be applied in other therapeutic and diagnostic procedures, both in the heart and in other organs.

### SYSTEM DESCRIPTION

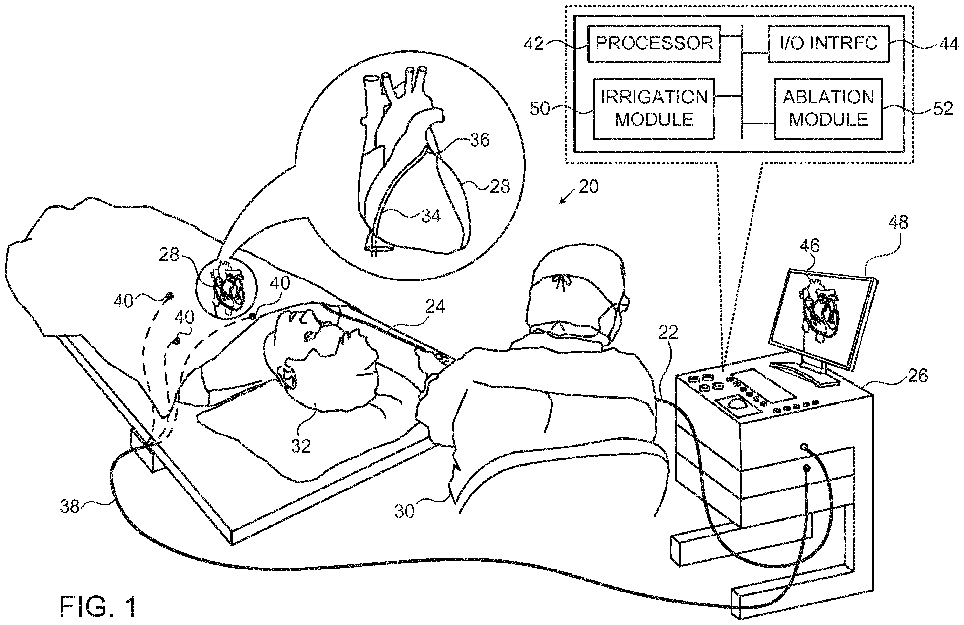

Figure 1 is a schematic, pictorial illustration of a medical system 20 configured to perform an ablation procedure,
in accordance with an embodiment of the present invention. System 20 comprises a probe 22, in the present example an intracardiac catheter comprising a flexible insertion tube 24, and a control console 26. Probe 22 is typically connected by a suitable connector at its proximal end to console 26.

In the embodiment described hereinbelow, it is assumed that probe 22 is used for diagnostic or therapeutic treatment, such as mapping electrical potentials of a heart 28, or performing ablation of endocardial tissue of the heart. Alternatively, probe 22 may be used, *mutatis mutandis,* for other therapeutic and/or diagnostic purposes in the heart or in other body organs.

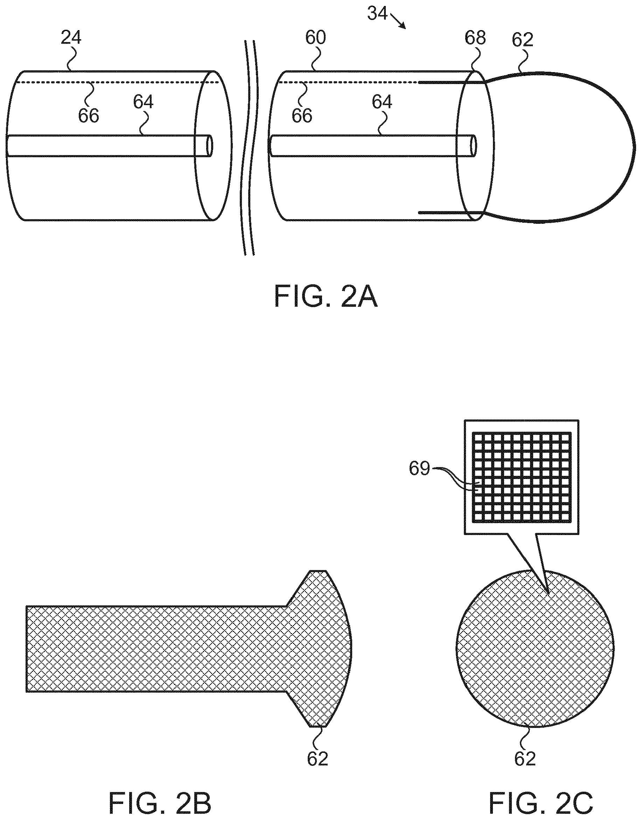

An operator 30, such as a cardiologist, inserts probe 22 through the vascular system of a patient 32 so that a distal end 34 of probe 22 enters a chamber of the patient's heart 28 (e.g., the left atrium). Operator 30 advances probe 22 so that a distal tip 36 of the probe engages body tissue at desired locations. As described in Figures 2A-3C hereinbelow, distal tip 36 comprises a deformable head 62, also referred to herein as a deformable distal end, comprising a flexible and porous material that may be conductive. In some embodiments deformable head 62 is conductive and can function as a position sensor, as described hereinbelow. In additional configurations, deformable head 62 is configured to convey radio frequency (RF) energy to intracardiac tissue of heart 28 during an ablation procedure.

In the example of Figure 1, console 26 is connected, via a cable 38, to body surface electrodes, which typically comprise adhesive skin patches 40 that are affixed to patient 32. Console 26 determines position coordinates of probe 22 inside heart 28 based on the impedance measured between the deformable head, when it is conductive, and patches 40. Although system 20 uses impedance-based sensing to measure a location of deformable head 62, other position tracking techniques may be used (e.g., magnetic-based sensors). Magnetic position tracking techniques are described, for example, in U.S. Patents 5,391,199, 5,443,489, 6,788,967, 6,690,963, 5,558,091, 6,172,499 6,177,792. Impedance-based position tracking techniques are described, for example, in U.S. Patents 5,983,126, 6,456,864 and 5,944,022.

Console 26 comprises a processor 42, which typically comprises a general-purpose computer, with suitable front end and interface circuits for receiving signals from probe 22 and controlling the other components of console 26. An input/output (I/O) communications interface 44 enables console 26 to interact with probe 22 and patches 40. Based on the signals received from probe 22 and from patches 40, processor 42 produces and displays a map 46 showing the position of distal tip 36 in the patient's body, the distance and/or contact indication between the loop and the body tissue, as well as status information and guidance regarding the procedure that is in progress. Map 46 is presented to operator 30 using a display 48. The position of probe 22 may be superimposed on map 46 or on another image of heart 28.

Processor 42 typically comprises a general-purpose computer, with suitable front end and interface circuits for receiving signals from probe 22 and controlling the other components of console 26. Processor 42 may be programmed in software to carry out the functions that are described herein. The software may be downloaded to console 26 in electronic form, over a network, for example, or it may be provided on non-transitory tangible media, such as optical, magnetic or electronic memory media. Alternatively, some or all of the functions of processor 42 may be carried out by dedicated or programmable digital hardware components.

Console 26 also comprises an irrigation module 50 and an RF ablation module 52. Processor 42 uses the ablation module to monitor and control ablation parameters such as the level of ablation power applied via deformable head 62. The ablation module may also monitor and control the duration of the ablation that is provided.

Typically, during ablation, heat is generated in the electrode (or electrodes) providing the ablation, as well as in the surrounding region. In order to dissipate the heat and to improve the efficiency of the ablation process, system 20 supplies an irrigation fluid to distal end 34 via a channel (described hereinbelow). System 20 uses irrigation module 50 to monitor and control irrigation parameters, such as the pressure, flow rate, and temperature of the irrigation fluid.

### CATHETER WITH A DEFORMABLE HEAD

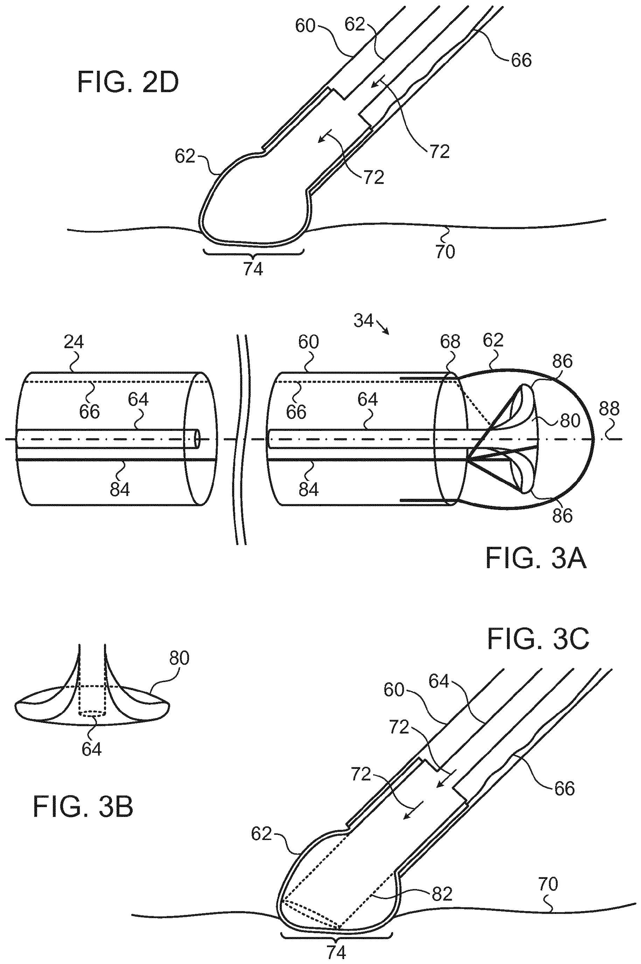

Figure 2A schematically illustrates distal end 34 of probe 22, Figures 2B and 2C are pictorial illustrations of a woven fabric used to construct deformable head 62, and Figure 2D is a schematic pictorial illustration of deformable head 62 in contact with endocardial tissue 70 of heart 28, in accordance with a first example.

Distal end 34 is covered by a flexible, insulating sheath 60 having a distal tip 68, and deformable head 62 is fixed to the distal end. In operation, deformable head 62 can be inflated and irrigated by a saline solution, which irrigation module 50 pumps through a channel 64, typically a tube, within sheath 60. An electrical conductor 66 passes within the catheter sheath to terminate at the deformable head and to convey radio-frequency (RF) electrical energy to deformable head 62.

Deformable head 62 may be made out of any suitable porous, flexible material. For some applications, a resilient, woven fabric may be advantageous. For enhanced mechanical strength and resilience, in one embodiment of the present invention, the fabric may be woven at least partially from elastic metal fibers, such as strands of Nitinol. This sort of implementation is illustrated in Figures 2B and 2C, which respectively show side and end views of deformable head 62. As described hereinbelow, the use of a metal-based fabric is also helpful in conducting electrical energy to the intracardiac tissue.

In the example shown in Figure 2D, irrigation module 50 conveys a saline solution 72 (or any other type of irrigation fluid) through channel 64, thereby generating a mechanical force sufficient to inflate deformable head 62 and to irrigate the tissue via pores 69 (Fig. 2C) in the porous and flexible material used to fabricate the deformable head. While deformable head 62 is inflated and pressed against endocardial tissue 70, the deformable head conforms to the endocardial tissue, as shown in the Figure.

In some embodiments, upon initially engaging endocardial tissue 70, deformable head 62 has an initial contact area 74 that comprises a portion of the flexible material that is in contact with the endocardial tissue. As deformable head 62 presses against endocardial tissue 70, contact area 74 can increase (up to a maximum contact area) as the deformable head deforms by spreading out.

As described supra, during insertion of tube 24, the deformable head need not be inflated. Therefore, sheath 60 may have a sheath diameter, and upon being inflated, deformable head 62 may have a head diameter that is greater than the sheath diameter. Typically, deformable head 62 has a shape that forms a seal between the deformable head and sheath 60. In some embodiments, deformable head 62 has a "balloon"-like shape.

When deformable head 62 is conductive, e.g., comprises suitable metal strands or a conductive polymer, ablation module 52 can convey RF energy to the deformable head via electrical conductor 66, and the deformable head conducts the energy to the tissue. Alternatively or additionally, electrical conductor 66 may apply the RF energy to saline solution 72, in which case the saline solution may conduct the RF energy through deformable head 62 to the endocardial tissue.

Figure 3A schematically illustrates distal end 34 of probe 22 comprising a wire frame 80, Figure 3B is a schematic pictorial illustration of the wire frame, and Figure 3C is a schematic pictorial illustration of deformable head 62 in contact with endocardial tissue 70 of heart 28, in accordance with an embodiment of the present invention. In Figures 3A and 3B, wire frame 80 is "mushroom"-shaped, coupled to electrical conductor 66, and affixed to a distal end of channel 64. In Figure 3C, a wire frame 82 is cylindrical, is coupled to electrical conductor 66, and is affixed to a distal end of sheath 60.

In embodiments of the present invention, deformable distal end 62 can be expanded and contracted by resizing wire frame 80. In the configuration shown in Figure 3A, the diameter of the mushroom-shaped wire frame can be resized via a control wire 84 that passes within the catheter sheath and is coupled to outer edges 86 of the wire frame. For example, if the mushroom-shaped wire frame is flexible, and operator 30 pulls on control wire 84, then the control wire can contract wire frame 80 by retracting outer edges 86 toward a longitudinal axis 88 of sheath 60. Likewise, if operator 30 pushes on control wire 84, the control wire can expand wire frame 80 by protracting outer edges 86 away from the longitudinal axis of the sheath.

While the examples in Figures 3A-3C show the wire frames in mushroom and cylindrical shapes, other shapes are considered to be within the scope of the present invention. Additionally, while the description hereinbelow describes configuration and operation of wire frame 80, wire frame 82 can be configured and operated in the same manner.

In this embodiment of the present invention, probe 22 comprises wire frame 80 that protrudes from sheath 60 and is covered by deformable distal head 62. Wires of frame 80 are flexible but resilient enough so that the frame maintains its overall form under deformation, as shown in Figure 3C. Wire frame 80 thus provides some mechanical stability to deformable head 62, allowing a more flexible fabric to be used for the deformable head.

As shown in Figure 3A, wire frame 80 extends from distal sheath tip 68 of sheath 60 and is contained within deformable head 62. Conductor 66 runs through sheath 60 and is coupled to wire frame 80. During an ablation procedure, conductor 66 conveys RF energy from ablation module 52 to wire frame 80, and the wire frame conveys the RF energy to endocardial tissue 70 when the wire frame presses against deformable head 62 and the deformable head is in contact with the endocardial tissue, as shown in Figure 3C. In an alternative embodiment, wire frame 80 can convey the RF energy to saline solution 72 (i.e., within deformable head 62) without the frame contacting head 62. In this case the saline solution may convey at least a portion of the RF energy to tissue 70.

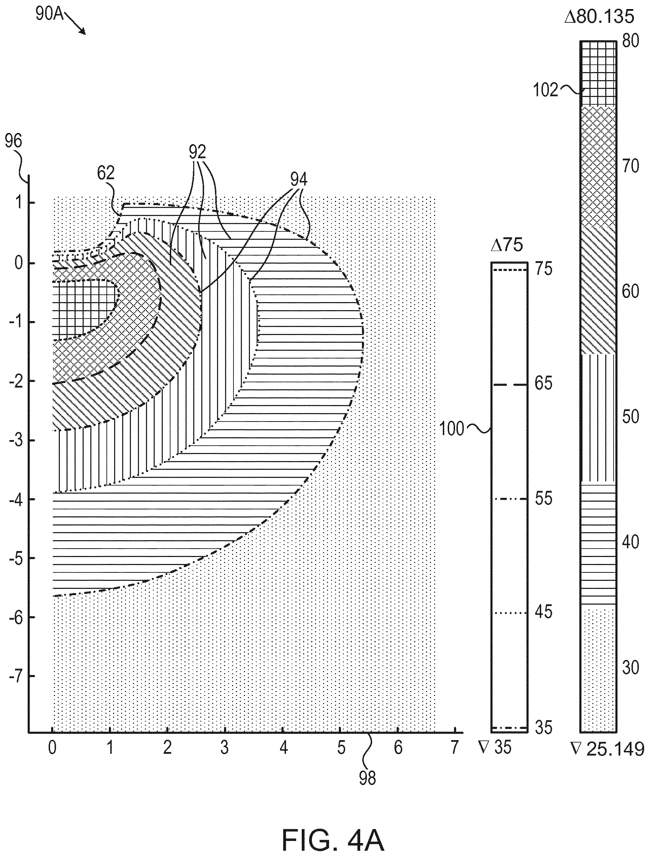

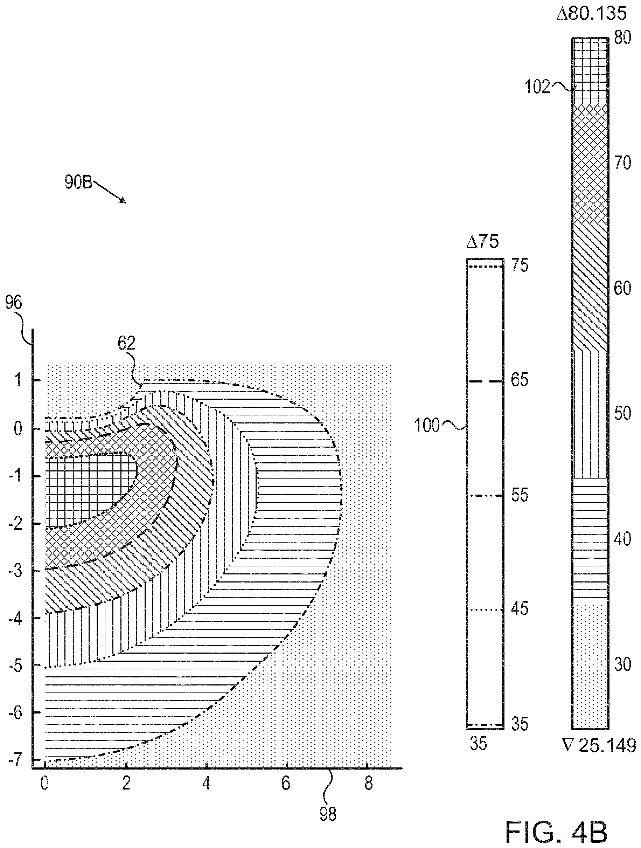

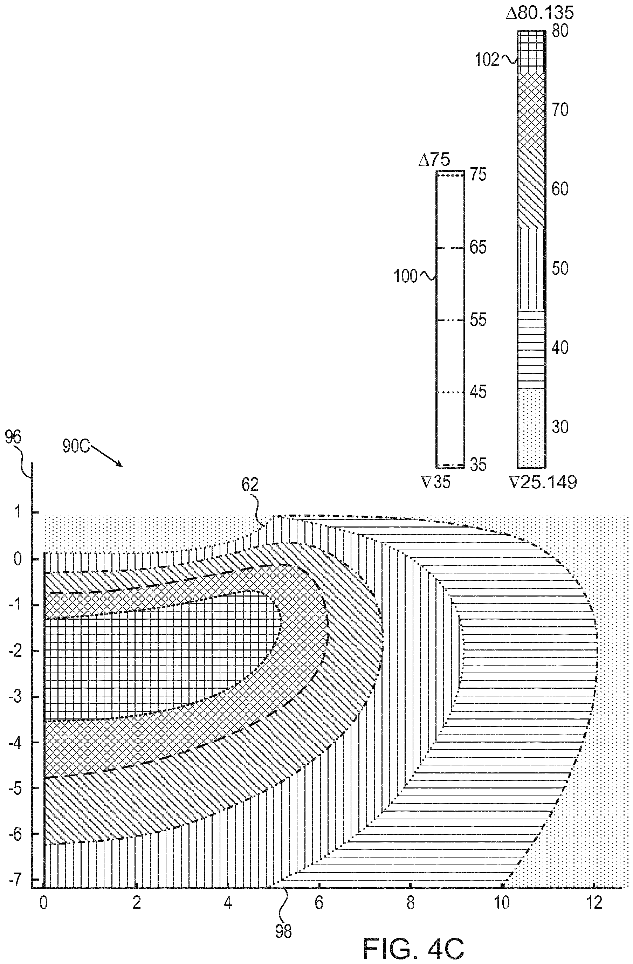

Figures 4A-4C show heat maps 90A-90C that illustrate one of the benefits of the deformable catheter head in terms of forming deeper and wider ablation lesions, by increasing an area of contact between the catheter head and the tissue (i.e., as deformable head 62 presses against endocardial tissue 70, as shown in Figures 2D and 3C). Figures 4A-4C show heat maps 90 (i.e., a heat map 90A in Figure 4A, a heat map 90B in Figure 4B and a heat map 90C in Figure 4C) that indicate how energy (i.e., heat) diffuses into endocardial tissue 70 during an ablation procedure using probe 22.

In the heat maps, a Y-axis 96 indicates a depth of endocardial tissue 70 relative to deformable head 62, and an X-axis 98 indicates a transverse displacement from deformable head 62 as the deformable head presses against the endocardial tissue. In Y-axis 96, a zero value indicates that deformable head 62 is in contact with endocardial tissue 70 while causing a depression, of approximately 1 mm, in the tissue.

The heat maps convey, using different visual patterns, indicated in a legend 102, a calculated tissue temperature during ablation as a function of depth (i.e., Y-axis 96) and transverse displacement (i.e., X-axis 98) relative to a center of the distal tip, which is located at the origin (0,0) at the left side of each figure. As shown in this visual representation, the hottest temperatures are measured at the left side of each figure below the catheter tip, and the coolest temperatures are measured at areas distant from the catheter tip, with intermediate temperatures measured in regions between the hottest and the coolest temperatures.

While for purposes of simplicity, the heat maps show regions 92 with uniform temperatures, in practice the temperature changes are typically gradual between isothermal lines 94, where each line style references a specific temperature, as indicated in a legend 100.

In some embodiments of the present invention, the contact area between distal tip 36 and endocardial tissue 70 can be controlled and increased as appropriate by suitably inflating and deforming the deformable head against the tissue. In the examples shown in Figures 4A-4C, the diameter of the contact area is increased from about 3 mm in Figure 4A, to about 5 mm in Figure 4B, to about 10 mm in Figure 4C. Additionally, the voltage on the catheter is increased from 25 V in Figure 4A, to 30 V in Figure 4B, to 35 V in Figure 4C, in order to keep the current density constant, and thus to maintain a constant temperature at the hottest spot in the tissue below the catheter. As shown in the heat maps, the width of the resulting lesions increases linearly with the distal tip contact area, while the depth increases, as well, though less markedly.

It will be appreciated that the embodiments described above are cited by way of example, and that the present invention is not limited to what has been particularly shown and described hereinabove. Rather, the scope of the present invention includes both combinations and subcombinations of the various features described hereinabove, as well as variations and modifications thereof which would occur to persons skilled in the art upon reading the foregoing description and which are not disclosed in the prior art.

## Claims

1. A medical probe (22), comprising:
a flexible insertion tube (24) having a deformable distal end (62) for insertion into a body cavity of a patient, the deformable distal end (62) comprising a flexible and porous material configured to be brought into contact with tissue (70) in the body cavity;
a means (50, 80, 82) for inflating the deformable distal end (62);
a channel (64) contained within the insertion tube (24) and configured to convey a fluid that irrigates the tissue (70) through pores (69) of the deformable distal end (62); and
an electrical conductor (66) passing through the flexible insertion tube (24) and terminating in the deformable distal end (62) and configured to convey radio frequency (RF) energy to the tissue (70) via the deformable distal end.
and wherein the means (80, 82) for inflating the deformable distal end (62) comprises a wire frame (80, 82) protruding from a distal tip (68) of the flexible insertion tube (24) and covered by the deformable distal end (62), and a control wire (84), passing through the flexible insertion tube (24), coupled to the wire frame (80, 82) and configured to resize the wire frame (80, 82), wherein distal movement of the control wire (84) is configured to expand the wire frame (80, 82), and further wherein proximal movement of the control wire (84) is configured to contract the wire frame (80, 82).

2. The medical probe (22) according to claim 1, wherein the flexible and porous material comprises a conductive material, and wherein the electrical conductor (66) is coupled to the flexible and porous material so as to convey the RF energy to the deformable distal end (62), and wherein conveying the RF energy to the tissue (70) comprises the deformable distal end (62) conveying the RF energy to the tissue (70).

3. The medical probe (22) according to claim 2, wherein the conductive material comprises a fabric woven from strands of Nitinol.

4. The medical probe (22) according to claim 2, wherein the conductive material is configured to transfer a current, the probe (22) further comprising a processor (42) configured to determine a position of the distal end (62) in response to an impedance to the current.

5. The medical probe (22) according to claim 1, wherein the fluid comprises a saline solution (72).

6. The medical probe (22) according to claim 5, wherein the electrical conductor (66) is configured to convey the RF energy to the saline solution (72), and wherein conveying the RF energy to the tissue (70) comprises the saline solution (72) conveying the RF energy to the tissue (70).

7. The medical probe (22) according to claim 1, wherein the probe (22) comprises an intracardiac catheter, and wherein the body cavity comprises a chamber of a heart (28) .

8. The medical probe according to claim 1, wherein the deformable distal end is configured to conform to the tissue of the body cavity.

9. The medical probe (22) according to claim 1, wherein the insertion tube (24) has a first diameter, and wherein upon inflation of the deformable distal end (62), the deformable distal end (62) has a second diameter greater than the first diameter.

10. The medical probe (22) according to claim 1, wherein a contact area between the deformable distal end (62) and the tissue (70) increases upon pressing the deformable distal end (62) against the tissue (70).

11. The medical probe (22) according to claim 1, wherein the electrical conductor (66) terminates in the wire frame (80, 82), and wherein the wire frame (80, 82) is configured to convey the RF energy from the electrical conductor (66) to the deformable distal end (62).

## Patentansprüche

1. Medizinische Sonde (22), umfassend
ein flexibles Einführrohr (24) mit einem verformbaren distalen Ende (62) zum Einführen in einen Körperhohlraum eines Patienten, wobei das verformbare distale Ende (62) ein flexibles und poröses Material umfasst, das ausgelegt ist, um in Kontakt mit Gewebe (70) in dem Körperhohlraum zu kommen;
ein Mittel (50, 80, 82) zum Befüllen des verformbaren distalen Endes (62);
einen Kanal (64), der in dem Einführrohr (24) enthalten ist und ausgelegt ist, um eine Flüssigkeit zu transportieren, welche das Gewebe (70) durch Poren (69) des verformbaren distalen Endes (62) spült; und
einen elektrischen Leiter (66), der das flexible Einführrohr (24) durchläuft und in dem verformbaren distalen Ende (62) endet und ausgelegt ist, um über das verformbare distale Ende Hochfrequenz- (HF)-Energie zu dem Gewebe (70) zu transportieren,
und wobei das Mittel (80, 82) zum Befüllen des verformbaren distalen Endes (62) einen Drahtrahmen (80, 82), der aus einer distalen Spitze (68) des flexiblen Einführrohrs (24) ragt und durch das verformbare distale Ende (62) bedeckt wird, und einen Steuerdraht (84) umfasst, der das flexible Einführrohr (24) durchläuft, an den Drahtrahmen (80, 82) gekoppelt ist und ausgelegt ist, um die Größe des Drahtrahmens (80, 82) zu ändern, wobei distale Bewegung des Steuerdrahts (84) ausgelegt ist, den Drahtrahmen (80, 82) zu expandieren, und wobei ferner proximale Bewegung des Steuerdrahts (84) ausgelegt ist, um den Drahtrahmen (80, 82) zu kontrahieren.

2. Medizinische Sonde (22) nach Anspruch 1, wobei das flexible und poröse Material ein leitfähiges Material umfasst, und wobei der elektrische Leiter (66) an das flexible und poröse Material gekoppelt ist, um so die HF-Energie zu dem verformbaren distalen Ende (62) zu transportieren, und wobei Transportieren der HF-Energie zu dem Gewebe (70) umfasst, dass das verformbare distale Ende (62) die HF-Energie zu dem Gewebe (70) transportiert.

3. Medizinische Sonde (22) nach Anspruch 2, wobei das leitfähige Material ein Textilmaterial umfasst, das aus Nitinol-Strängen gewebt ist.

4. Medizinische Sonde (22) nach Anspruch 2, wobei das leitfähige Material ausgelegt ist, um einen Strom zu übertragen, wobei die Sonde (22) ferner einen Prozessor (42) umfasst, der ausgelegt ist, um eine Position des distalen Endes (62) in Reaktion auf eine Impedanz zu dem Strom zu bestimmen.

5. Medizinische Sonde (22) nach Anspruch 1, wobei die Flüssigkeit eine Kochsalzlösung (72) umfasst.

6. Medizinische Sonde (22) nach Anspruch 5, wobei der elektrische Leiter (66) ausgelegt ist, um die HF-Energie zu der Kochsalzlösung (72) zu transportieren, und wobei Transportieren der HF-Energie zu dem Gewebe (70) umfasst, dass die Kochsalzlösung (72) die HF-Energie zu dem Gewebe (70) transportiert.

7. Medizinische Sonde (22) nach Anspruch 1, wobei die Sonde (22) einen intrakardialen Katheter umfasst, und wobei der Körperhohlraum eine Kammer eines Herzens (28) umfasst.

8. Medizinische Sonde nach Anspruch 1, wobei das verformbare distale Ende ausgelegt ist, um sich an das Gewebe des Körperhohlraums anzuschmiegen.

9. Medizinische Sonde (22) nach Anspruch 1, wobei das Einführrohr (24) einen ersten Durchmesser aufweist, und wobei das verformbare distale Ende (62) nach Befüllen des verformbaren distalen Endes (62) einen zweiten Durchmesser aufweist, der größer als der erste Durchmesser ist.

10. Medizinische Sonde (22) nach Anspruch 1, wobei ein Kontaktbereich zwischen dem verformbaren distalen Ende (62) und dem Gewebe (70) wächst, wenn das verformbare distale Ende (62) gegen das Gewebe (70) gepresst wird.

11. Medizinische Sonde (22) nach Anspruch 1, wobei der elektrische Leiter (66) in dem Drahtrahmen (80, 82) endet, und wobei der Drahtrahmen (80, 82) ausgelegt ist, um die HF-Energie von dem elektrischen Leiter (66) zu dem verformbaren distalen Ende (62) zu transportieren.

## Revendications

1. Sonde médicale (22), comprenant :
un tube d'insertion flexible (24) possédant une extrémité distale déformable (62) pour l'insertion dans une cavité corporelle d'un patient, l'extrémité distale déformable (62) comprenant un matériau flexible et poreux configuré pour être mis en contact avec un tissu (70) dans la cavité corporelle ;
un moyen (50, 80, 82) pour gonfler l'extrémité distale déformable (62) ;
un canal (64) contenu à l'intérieur du tube d'insertion (24) et configuré pour transporter un fluide, qui arrose le tissu (70), à travers des pores (69) de l'extrémité distale déformable (62) ; et
un conducteur électrique (66) passant à travers le tube d'insertion flexible (24) et se terminant dans l'extrémité distale déformable (62) et configuré pour transporter de l'énergie à radiofréquence (RF) vers le tissu (70) par l'intermédiaire de l'extrémité distale déformable, et dans laquelle le moyen (80, 82) pour gonfler l'extrémité distale déformable (62) comprend un cadre de fils (80, 82) faisant saillie à partir d'un embout distal (68) du tube d'insertion flexible (24) et couvert par l'extrémité distale déformable (62), et un fil de commande (84), passant à travers le tube d'insertion flexible (24), couplé au cadre de fils (80, 82) et configuré pour redimensionner le cadre de fils (80, 82), dans laquelle le mouvement distal du fil de commande (84) est configuré pour agrandir le cadre de fils (80, 82), et en outre dans laquelle le mouvement proximal du fil de commande (84) est configuré pour contracter le cadre de fils (80, 82).

2. Sonde médicale (22) selon la revendication 1, dans laquelle le matériau flexible et poreux comprend un matériau conducteur, et dans laquelle le conducteur électrique (66) est couplé au matériau flexible et poreux afin de transporter l'énergie RF vers l'extrémité distale déformable (62), et dans laquelle le transport de l'énergie RF vers le tissu (70) comprend le transport, par l'extrémité distale déformable (62), de l'énergie RF vers le tissu (70).

3. Sonde médicale (22) selon la revendication 2, dans laquelle le matériau conducteur comprend un tissu tissé à partir de brins de Nitinol.

4. Sonde médicale (22) selon la revendication 2, dans laquelle le matériau conducteur est configuré pour transférer un courant, la sonde (22) comprenant en outre un processeur (42) configuré pour déterminer une position de l'extrémité distale (62) en réponse à une impédance au courant.

5. Sonde médicale (22) selon la revendication 1, dans laquelle le fluide comprend une solution saline (72).

6. Sonde médicale (22) selon la revendication 5, dans laquelle le conducteur électrique (66) est configuré pour transporter l'énergie RF vers la solution saline (72), et dans laquelle le transport de l'énergie RF vers le tissu (70) comprend le transport, par la solution saline (72), de l'énergie RF vers le tissu (70).

7. Sonde médicale (22) selon la revendication 1, dans laquelle la sonde (22) comprend un cathéter intracardiaque, et dans laquelle la cavité corporelle comprend une chambre d'un coeur (28).

8. Sonde médicale selon la revendication 1, dans laquelle l'extrémité distale déformable est configurée pour épouser le tissu de la cavité corporelle.

9. Sonde médicale (22) selon la revendication 1, dans laquelle le tube d'insertion (24) possède un premier diamètre, et dans laquelle, lors du gonflement de l'extrémité distale déformable (62), l'extrémité distale déformable (62) possède un second diamètre supérieur au premier diamètre.

10. Sonde médicale (22) selon la revendication 1, dans laquelle une zone de contact entre l'extrémité distale déformable (62) et le tissu (70) augmente lors de la pression de l'extrémité distale déformable (62) contre le tissu (70) .

11. Sonde médicale (22) selon la revendication 1, dans laquelle le conducteur électrique (66) se termine dans le cadre de fils (80, 82), et dans laquelle le cadre de fils (80, 82) est configuré pour transporter l'énergie RF à partir du conducteur électrique (66) vers l'extrémité distale déformable (62).
